# EUROPÄISCHE PATENTSCHRIFT

(11) **EP 3 970 705 B1**
(45) Veröffentlichungstag und Bekanntmachung des Hinweises auf die Patenterteilung: **16.08.2023**
(21) Anmeldenummer: 21198189.9
(22) Anmeldetag: 22.09.2021
(51) Int. Cl.: A61K 9/70, A61K 47/24, A61K 47/26, A61K 47/40, A61K 31/352

(54) **FILMBILDENDE SPRÜHPFLASTER ZUR DERMALEN UND TRANSDERMALEN APPLIKATION VON STOFFEN ENTHALTEND KOMPLEXIERENDE HILFSSTOFFE ZUR MOLEKULAREN KOMPLEXIERUNG**
FILM FORMING SPRAY PLASTERS FOR DERMAL AND TRANSDERMAL APPLICATION OF SUBSTANCES CONTAINING COMPLEXING ADJUVANTS FOR MOLECULAR COMPLEXATION
PANSEMENT PAR PULVÉRISATION FILMOGÈNE POUR L'APPLICATION DERMIQUE ET TRANSDERMIQUE DE SUBSTANCES CONTENANT DES ADDITIFS COMPLEXANTS DESTINÉS À LA COMPLEXATION MOLÉCULAIRE

(30) Priorität: 22.09.2020 DE 202020003998 U
(43) Veröffentlichungstag der Anmeldung: 23.03.2022
(73) Patentinhaber: Inclusion GmbH, 13595 Berlin (DE)
(72) Erfinder: TACK, Johannes, 13595 Berlin (DE)
(74) Vertreter: Seuss, Thomas

(56) Entgegenhaltungen:
- WO-A2-2010/029093
- US-A1- 2007 248 658
- US-A1- 2016 361 271
- US-A1- 2017 296 485
- KARANDE P ET AL: "Enhancement of transdermal drug delivery via synergistic action of chemicals", BIOCHIMICA ET BIOPHYSICA ACTA, ELSEVIER, AMSTERDAM, NL, Bd. 1788, Nr. 11, 1. November 2009 (2009-11-01), Seiten 2362-2373, XP026708909, ISSN: 0005-2736, DOI: 10.1016/J.BBAMEM.2009.08.015 [gefunden am 2009-09-03]
- MARQUES HELENA MA CABRAL: "A review on cyclodextrin encapsulation of essential oils and volatiles", FLAVOUR AND FRAGRANCE JOURNAL., Bd. 25, Nr. 5, 1. September 2010 (2010-09-01), Seiten 313-326, XP055883622, GB ISSN: 0882-5734, DOI: 10.1002/ffj.2019

## Beschreibung

Die Erfindung betrifft Sprühpflaster, welche durch die Einarbeitung von komplexierenden Hilfsstoffen in die Sprühlösung die Applikation von Cannabidiol und Tetrahydrocannabidiol auf der Haut ermöglicht.

Sprühpflaster sind allgemein bekannt und für unterschiedliche Anwendungen bereits im Gebrauch. So sind wirkstofffreie Sprühpflaster als moderner Pflasterersatz bekannt, bei denen die gelösten Polymere nach dem Verdampfen der flüchtigen Lösemittel auf der Haut eine Schutzschicht bilden, eine Wunde vor Wasser, Schmutz und Mikroorganismen schützen und dadurch das Anlegen eines Verbandes erübrigen.

Auch bekannt sind Sprühpflaster, welche neben den konstituierenden Polymeren zusätzlich pharmazeutische Wirkstoffe gelöst oder suspendiert enthalten, um deren therapeutischen Effekte an der intakten Haut oder nach Resorption durch die Haut in der system ischen Zirkulation zur Wirkung zu bringen. Derartige Sprühpflaster können zusätzliche Hilfsstoffe enthalten um, die Permeation der Wirkstoffe durch die Haut zu verstärken und auch deren Freigabe verzögert über eine längere Zeit nach der Applikation zu erreichen.

So beschreibt US 4303066A Sprühpflaster, welche durch geeignete Zusätze von Lösungsvermittlern hydrophile Polymerfilme zur Wundversorgung ermöglichen.

Weitere Sprühpflaster sind mit den Anmeldungen DE102008060904A1, DE 2343923 und DE 2924042 bekannt geworden, wobei in filmbildenden hydrophoben Polymeren durch Zugabe von Lösungsvermitteln die Einarbeitung von Wirkstoffen ermöglicht wird. Die Verwendung von Antiseptika wie Menthol, Thymol, Eugenol wird dadurch zwar technisch möglich aber wegen der hohen Flüchtigkeit dieser Verbindungen als problematisch angesehen, da die notwendigen Konzentrationen für die antiseptische Wirksamkeit nicht erreicht werden.

In der Anmeldung EP 1 654 013 B1, welche sich hauptsächlich mit antimikrobiellen Silberverbindungen in selbstklebenden Wundauflagen befasst, wird Menthol und Cyclodextrin erwähnt, aber die Ausführung in Form einer Beispielrezeptur wird nicht offengelegt und die Verwendung von Cyclodextrinen auch nicht beansprucht. Ebenso wird in der Anmeldung DE20221244 U1, welche ein konventionelles Transdermales Pflaster mit Backingfolie und klebender Polymermatrix beschreibt, die molekulare Verkapselung von Menthol mit Cyclodextrinen beschrieben, allerdings ebenfalls ohne ein konkretes Ausführungsbeispiel, welches die Erfindung offenlegen würde und ohne Beanspruchung von Cyclodextrin bzw. dessen Derivate.

Die Anmeldung WO2007077029A1 beschreibt wirkstoffhaltige Sprühpflaster auf der Basis von Polyurethan- und Polyacrylat-Polymermischungen aus der Haarpflege, welche durch Zusatz von Lösungsvermittlern und Permeationsverstärkern die Einarbeitung von pharmazeutischen Wirkstoffen ermöglicht. Eine Stabilisierung und gesteuerte Freisetzung von empfindlichen, leicht flüchtigen Wirkstoffen wie z.B. Terpene kann nur in begrenztem Masse erreicht werden.

Diese Nachteile werden als umso problematischer empfunden, weil durch die beschriebenen Beschränkungen der bisherigen technischen Lösungen eine große Anzahl von Wirkstoffen, Arzneistoffen und Aromastoffen, die für die Anwendung am Menschen vorteilhaft sein können, nicht auf diesem Weg zu Anwendung kommen bzw. von der außerordentlich anwendungsfreundlichen Applikation mittels Sprühpflaster profitieren können.

Im Gegensatz dazu beschreibt US 2016/361271 A1 transdermale Verabreichungsvorrichtungen für die Verabreichung von Cannabidiol (CBD) mit einem "Reservoir-Pflaster-Design" gebildet aus einer mikroporösen, hydrophilen Membran und einem Reservoir, das eine Mischung aus CBD, einer polaren Flüssigkeit und einem Geliermittel enthält. Die hydrophile Membran ist mit einem aminkompatiblen Silikon-Hautkleber beschichtet.

Der Erfindung liegt daher die Aufgabe zugrunde, ein Sprühpflaster zu schaffen, welches die oben beschriebenen Nachteile bekannter Sprühpflasterapplikationen weitgehend beseitigt.

Diese Aufgabe wird durch das vorgestellte Sprühpflaster gelöst, deren Formulierung zusätzlich zu den Wirkstoffen, Arzneistoffen und Aromastoffen komplexierende Hilfsstoffe enthält, die sich einerseits durch ihre Fähigkeit geeignete Stoffe molekular zu komplexieren bzw. zu verkapseln auszeichnen und andererseits so ausgewählt sind, dass sie mit in den bekannten Prozessstoffen bzw. Lösemitteln, die für die Herstellung von Sprühpflastern verwendet werden, kompatibel und prozessierbar sind.

Die Erfindung betrifft daher ein Wirkstoff enthaltendes Sprühpflaster, enthaltend mindestens ein bei Raumtemperatur leicht verdampfbares Lösungsmittel, mindestens einen pharmazeutischen Wirkstoff, ausgewählt aus Cannabidiol und Tetrahydrocannabidiol,
mindestens ein Acrylpolymer und/oder ein Urethanpolymer, welches nach Verdampfen des Lösemittels einen transparenten, atmungsaktiven Film auf der Haut bildet,
wobei das Acrylpolymer ausgewählt aus Methylprop-2-enoic acid-N,N-dimethylmethanamine (2/1) (Eudragit^{®} RL/RS) oder ein anionisches Copolymer mit Vinylacetat/Crotonsäure (Luviset CA-66) ist, welches mit geeigneten Aminoalkanolen vorzugsweise mit Aminomethylpropanol neutralisiert ist
und wobei das Urethanpolymer ausgewählt ist aus Polyurethan-4 (Avalur UR 450), Polyurethan-6 (Avalur Flex-6) oder Polyurethane-14 und AMP-Acrylat Copolymer (DynamX)^{®} oder ein anionisches Polymer mit endständiger Säuregruppe ist (Luviset P.U.R.), welches mit organischen oder anorganischen Aminoalkoholen wie z.B. mit Triethanolamin neutralisiert ist,
**dadurch gekennzeichnet, dass** die Sprühlösung zusätzlich mindestens einen komplexierenden Hilfsstoff enthält, wobei als komplexierender Hilfsstoff α-, ß- oder y-Cyclodextrin, substituierte Cyclodextrine, Lecithine, Polysorbate, Glycocholsäure und/oder Pflanzenöle enthalten ist.

Das Sprühpflaster enthält zunächst konstituierende Polymere, welche aus einem Acrylpolymer und/oder einem Polyurethanpolymer bestehen entweder allein oder in einer geeigneten Mischung der Polymere.

Die Formulierung des Sprühpflasters kann auch anionische Polymere allein oder in Mischung enthalten, in Verbindung mit einem geeigneten Neutralisationsmittel, welche eine Reduktion der flüchtigen Bestandteile und eine Erhöhung des Anteils an Wasser oder hydrophilen Bestandteilen in der Formulierung ermöglicht.

Als besonders geeignete Acrylpolymere sind zu nennen:
Methylprop-2-enoic acid-N,N-dimethylmethanamine (2/1) (Eudragit^{®} RL/RS) oder ein anionisches Copolymer mit Vinylacetat/Crotonsäure (Luviset CA-66) ist, welches mit geeigneten Aminoalkanolen vorzugsweise mit Aminomethylpropanol neutralisiert ist.

Als besonders geeignete Urethanpolymere sind zu nennen:
Polyurethan-4 (Avalur UR 450), Polyurethan-6 (Avalur Flex-6) oder Polyurethane-14 und AMP-Acrylat Copolymer (DynamX)^{®}
oder ein anionisches Polymer mit endständiger Säuregruppe ist (Luviset P.U.R.), welches mit organischen oder anorganischen Aminoalkoholen wie z.B. mit Triethanolamin neutralisiert ist.

Mischungen der genannten Acryl- und/oder Urethanpolymere sind möglich. Gegebenenfalls können zusätzlich übliche Weichmacher (z.B. Triethylcitrat, Triethylenglycol) verwendet werden. Der Gewichtsanteil der Weichmacher liegt üblicherweise im Bereich 0,5 bis 2,5% (w/w).

Die genannten Acryl- und/oder Urethanpolymere sind in mindestens einem bei Raumtemperatur leicht verdampfbaren Lösungsmittel gelöst. Als Lösungsmittel für diese Anwendung kommen Ethanol, Isopropanol, Ethylacetat, Aceton und deren Gemische in Betracht.

Der Prozess der Filmbildung des Sprühpflasters durch Verdampfen der Lösemittel ist in weiten Teilen analog zu den Verfahren, mit denen molekulare Komplexierungen / Verkapselungen effizient durchgeführt werden und hohe Einschlussraten erreicht werden
Komplexierende Hilfsstoffe mit denen sich molekulare Komplexierungen bzw. Verkapselungen durchführen lassen sind insbesondere Cyclodextrine entweder in der nativen Form als α-, ß- oder y-CD oder substituierte CDs wie (2-Hydroxypropyl-ß-Cyclodextrin (β-HPCD) oder 17ß-Hydroxybutenyl-CD, um nur beispielhaft eine Auswahl der Cyclodextrine zu benennen, die für das erfinderische Sprühpflaster geeignet sind. Zu anderen sind komplexierende Hilfsstoffe geeignet, welche die Bildung von Mizellen insbesondere von Mischmizellen (MiMi) ermöglichen. Mizellare Strukturen aus ambiphilen Substanzen oder Substanzgemischen sind in vergleichbarer Weise für den Einschluss von schwer wasserlöslichen und instabilen Substanzen geeignet und können überraschend in die Formulierung bzw. Applikation der erfinderischen Sprühpflaster integriert werden. Als komplexierende Hilfsstoffe für Mischmizellen seien beispielhaft Lecithine (z.B. Soja-Lecithin), Polysorbate, insbesondere Polysorbat 80 und Glycocholsäure genannt. Typischerweise sind diese komplexierenden Hilfsstoffe im molaren Verhältnis 1:1 bis 1:2 und im Konzentrationsbereich 2-15% (w/w) enthalten.

Als komplexierende Hilfsstoffe sind überraschenderweise auch Pflanzenöle, wie z.B. MCT-Öl, Kokosöl im Konzentrationsbereich 5-15% (w/w) geeignet. Diese Pflanzenöle sind in der Lage, nanoskalige Öl-in-Wasser-Emulsionen zu bilden, so dass sie als komplexierende Hilfsstoffe fungieren können.

Die vorab aufgeführten Hilfsstoffe finden seit langem in der Nahrungsmittel-, Kosmetik- und Pharmaindustrie Verwendung und sind für die beabsichtigte Verwendung als außerordentlich sicher einzustufen. Solubilisierung von schwerlöslichen Stoffen, Stabilisierung von empfindlichen Stoffen und die Verzögerung der Freisetzung von Stoffen bzw. permeationsverstärkende Wirkung an der Haut in den verschiedenen Anwendungsformen der genannten Industrien sind die allgemeine und verbreitete Anwendung.

So lässt sich die Empfindlichkeit gegen Licht, Sauerstoff und Luftfeuchtigkeit von Vanillin, Menthol, R-(+)-Limonen, Zimt- und Orangenöl in Form von CD-Komplexen verringern und die Haltbarkeit und Freisetzung an das Zielorgan verbessern bzw. verlängern. Lipidmoleküle wie Linolsäure, Retinol und α-Tocopherol sind als CD-Komplex besser gegen UV- Strahlen geschützt und geben zudem die Wirkstoffe kontrolliert aus dem Hohlraum der CD-Komplexe frei. Wasserunlösliche Wirkstoffe wie Konakion^{™} oder Benzodiazepine können durch Mischmizellen und CD-Komplexierung löslich gemacht werden, so dass therapeutische Dosierungen in kleinen Volumina parenteral als wässrige Lösung verabreicht werden können. Wasserunlösliche Wirkstoffe mit vergleichsweise niedrigem Schmelzpunkt oder amorpher Struktur (Cannabidiol, Tetrahydrocannabidiol) können durch Komplexierung für dermale und transdermale Verwendung formuliert werden, wobei die zur Komplexierung verwendeten Hilfsstoffe als Permeationsverstärker einen zusätzlichen Effekt auf den Flux durch die intakte Haut ausüben können. Als Permeationsverstärker können insbesondere das Natriumsalz der Ölsäure, Isopropylmyristat sowie Kombinationen dieser beiden Verbindungen im Konzentrationsbereich zwischen 0,5 bis 5% (w/w) eingesetzt werden.

Die erfindungsgemäßen Sprühpflaster können ferner feuchthaltende Hilfsstoffe enthalten, wie Glycerol, Sorbitol, Polyethylenglycol oder Polyvinylpyrrolidon sowie deren Mischungen im Konzentrationsbereich zwischen 0,25 und 10 % (w/w) und mit einem Gewicht pro Sprühstoß von 50 bis 150 mg (Feuchtgewicht).

Weitere optionale Komponenten der erfindungsgemäßen Formulierungen sind Aromastoffe wie Menthol, Vanillin, Zimt- und Orangenöl insbesondere R-(+)-Limonen sowie deren Kombinationen im Konzentrationsbereich 0,1 bis 10% ( w/w).

Überraschenderweise lassen sich diese vorteilhaften Eigenschaften der beschriebenen funktionalen Hilfsstoffe in einfacher Weise in die Formulierung von Sprühpflastern integrieren

Durch die vorliegende Erfindung wird für den Anwender auf einfache und anwenderfreundliche Weise die dermale bzw. transdermale Anwendung von empfindlichen und flüchtigen Stoffen möglich. Wirkstoffe, Arzneistoffe und Aromastoffe lassen sich durch Sprühpflaster einfach dosieren, in der Weise, dass die zur Anwendung verwendeten Pumpspraybehälter 25 - 200 mg der Sprühformulierung, vorzugsweise 50 - 100 mg mit einem Sprühstoß an die Haut abgeben und daran einen Feststoffanteil aus Wirkstoffen, Arzneistoffen, Aromastoffen und nicht flüchtigen Hilfsstoffen von 15 - 50% aufweisen. Die Hautfläche, die durch einen Sprühstoß in Anspruch genommen wird, beträgt in der Regel 10 bis 20 cm², je nach technischer Ausformung des Sprühventils.

Die transdermale Anwendung von Stoffen, insbesondere wenn der Formulierung des Sprühpflasters penetrationsfördernde Hilfsstoffe, welche auch identisch mit den beschriebenen komplexierenden Hilfsstoffen (Cyclodextrine, -derivate, Mischmizellen) sein können, zugesetzt sind, haben gegenüber der sonst üblichen oralen Anwendung der Stoffe sehr häufig den Vorteil einer signifikant verbesserten Bioverfügbarkeit. Stoffe, die nach oraler Anwendung nach der Absorption im Gastrointestinaltrakt einer erheblichen enzymatischen Inaktivierung während der ersten Leberpassage unterliegen, können nach transdermaler Gabe eine deutliche Verbesserung der Bioverfügbarkeit aufweisen, da die Stoffe bei der Penetration durch die intakte Haut direkt in die systemische Zirkulation aufgenommen werde. Als Beispiel sei die Verbesserung der Bioverfügbarkeit nach transdermaler Gabe von Cannabidiol um den Faktor 8-10 gegenüber der oralen bzw. peroralen Verabreichung genannt (s. Mitteilung der XPhyto Therapeutics, Vancouver, Kanada vom 11. November 2019). Ebenso kann bekanntlich die Bioverfügbarkeit von 17ß-Estradiol, welche nach peroraler Gabe ca. 2% der applizierten Dosis beträgt, durch transdermale Applikation um den Faktor 40-50 gesteigert werden.

Dadurch kann derselbe gewünschte Effekt der verabreichten Stoffe oft mit einer erheblich geringeren Dosis erreicht werden, welches die Sicherheit der Anwendung erhöht bzw. eine beabsichtigte oder versehentliche missbräuchliche Anwendung der Stoffe praktisch unmöglich macht.

Zudem kann die in einem Sprühpflaster die nach der Applikation enthaltende Dosis eines Stoffes bei Unverträglich leicht entfernt werden, was bei einer oralen Applikation nicht möglich ist.

### Beispiele

Nachstehend beschriebene Beispiele von sprühfähigen Lösungen illustrieren mögliche Sprühpflasterzusammensetzungen. Die prozentualen Gewichtsanteile beziehen sich auf die Gesamtmasse der Zubereitungen, Trägergase, die zusätzlich für die Anwendung als Druckspray zugesetzt werden können, sind dabei nicht berücksichtigt. Zusammensetzungen mit anderen Wirkstoffen als Cannabidiol und/oder Tetrahydrocannabidiol dienen nur der Erläuterung der Bandbreite möglicher Zusammensetzungsvarianten, sind jedoch nicht beansprucht.
1. Sprühfähige Lösung aus Ethanol 61%, Menthol 0,5%, R-(+)-Limonen 2%, β-HPCD 3%, 2-Methylprop-2-enoic acid--N,N-dimethylmethanamine (2/1) (Eudragit^{®} RL) 9,7%, Triethylenglycol 1,5%, Isopropylalkohol 16,1%, Wasser 6,2%
2. Sprühfähige Lösung aus Ethanol 64%, Nicotin 3,1%, β-HPCD 11,8%, Polyurethane-14 und AMP-Acrylat Copolymer (DynamX^{®}) 8,8%, Triethylcitrat 1,5%, Isopropanol 4,6%, Wasser 6,2%
3. Sprühfähige Lösung aus Ethanol 68%, Cannabidiol (CBD) 3,6 % (davon 0,6 % wasserlösliches mixed micelle, full spectrum CBD (10,4%)), β-HPCD 3,8%, Polyurethane-14 und AMP-Acrylat Copolymer DynamX^{®}) 12,8%, Triethylcitrat 1,5%, Glycerol 4,6%, Wasser 5,7%
4. Sprühfähige Lösung aus Ethanol 65%, Nitroglycerin 4,2%, β-HPCD 4,8%, Polyurethane-14 und AMP-Acrylat Copolymer (DynamX^{®}) 7,8%, 2-Methylprop-2-enoic acid--N,N-dimethylmethanamine (2/1) (Eudragit^{®} RL) 7%,Triethylcitrat 1,5%, Glycerol 3,6%, Wasser 6,1%.
5. Sprühfähige Lösung aus Ethanol 67%, 17ß Estradiol 2,4%, β-HPCD 11,8%, Polyurethan-1, (Luviset^{®} P.U.R.) 4,9%, 2-Methylprop-2-enoic acid-N,N-dimethylmethan-amine (2/1)(Eudragit ORL)5,9%,Triethylcitrat 2,0%, Polyethylenglycol 2,8%, Wasser 3,2%
6. Sprühfähige Lösung aus Ethanol 63,2%, Lisurid 2,1%, Povidone K90 5%, Polyurethane-14 und AMP-Acrylat Copolymer (DynamX)^{®} 9,8%, Triethylcitrat 2,0%, Polysorbat 80 6,4%, Soja-Lecithin 4,7%, Glycerol 3,6%, Wasser 3,2%
7. Sprühfähige Lösung aus Ethanol 67,3%, Cannabidiol (CBD Isolat) 4,1% und 0,8 % wasserlösliches mixed micelle, full spectrum CBD (10,4%)), Polyurethane-14 und AMP-Acrylat Copolymer DynamX^{®}) 9,4%, Triethylcitrat 1,5%, Polysorbat 80 4,2%, Soja-Lecithin 4,9%, Glycerol 4,7%, Wasser 3,9%
8. Sprühfähige Lösung aus Ethanol 63,9%, Testosteron 5,2%, Polyurethane-14 und AMP-Acrylat Copolymer (DynamX^{®}) 9,8%, Triethylcitrat 1,5%, Isopropylmyristat 3,1%, Polysorbat 80 5,9% Soja-Lecithin 8,4%, Wasser 2,2%
9. Sprühfähige Lösung aus Ethanol 69%, Cannabidiol (CBD) 1,6 % (davon 0,3 % wasserlösliches mixed micelle, full spectrum CBD (10,4%)), β-HPCD 4,8%, Polyurethane-14 und AMP-Acrylat Copolymer DynamX^{®}) 10,8%, Triethylcitrat 1,5%, Glycerol 5,6%, Wasser 6,7%
10. Sprühfähige Lösung aus Ethanol 67%, Rotigotin 10%, Povidone K90 5%, Polyurethane-14 und AMP-Acrylat Copolymer (DynamX^{®}) 8,7%, Triethylcitrat 1,5%, Glycerol 4,6%, Wasser 3,2%
11. Sprühfähige Lösung aus Ethanol 67%, 8-Prenylnaringenin 10%, Povidone K90 5%, Polyurethane-14 und AMP-Acrylat Copolymer (DynamX^{®}) 8,7%, Triethylcitrat 1,5%, Glycerol 4,6%, Wasser 3,2%
12. Sprühfähige Lösung aus Ethanol 46%, Cannabidiol 2,4%, β-HPCD 3,8%, Polyurethan-1, (Luviset^{®} CA-66) 7,9%, 2-Methylprop-2-enoic acid-N,N-dimethylmethan-amine (2/1)(Eudragit ^{®}RL) 8,9%,Triethylcitrat 2,0%, Polyethylenglycol 2,8%, Wasser 26,2%
13. Sprühfähige Lösung aus Ethanol 63,9%, Canabidiol (CBD) 3,2%, Melatonin 1,0% Polyurethane-14 und AMP-Acrylat Copolymer (DynamX^{®})11,8%, Triethylcitrat 1,5%, Isopropylmyristat 2,1%, Polysorbat 80 5,9% Soja-Lecithin 9,4%, Wasser 1,2%
14. Sprühfähige Lösung aus Ethanol 46%, Curcumin 2,4%, β-HPCD 3,8%, Polyurethan-1, (Luviset^{®} P.U.R) 7,9%, 2-Methylprop-2-enoic acid-N,N-dimethylmethan-amine (2/1)(Eudragit ^{®}RL) 8,9%,Triethylcitrat 2,0%, Polyethylenglycol 2,8%, Wasser 26,2%
15. Zusammensetzungen gemäß der Bespiele 1-14 werden jeweils unter Rühren bei 20°C -40°C unter Rühren gemischt. 10 ml der jeweils Lösung aus den Beispielen 1-14 werden in einen handelsüblichen Container aus Weißblech, Aluminium, Kunststoff oder Glas abgefüllt und mit einem handelsüblichen Pumpkopf (z.B. Suneast, 10ml Sprühflasche aus Braunglas (z.B. Apotheker Flasche mit Sprühkopf und Schutzkappen, Art.Nr. C20175, ean8043016063159). Durch manuellen Druck auf den Pumpkopf werden pro Hub (je nach Modell) 25 - 200 mg der Sprühformulierung abgegeben.
16. Zusammensetzungen gemäß der Bespiele 1-14 werden jeweils unter Rühren bei 20°C -40°C unter Rühren gemischt. 10 ml der jeweils Lösung aus den Beispielen 1-14 werden in einen handelsüblichen Container aus Weißblech abgefüllt und mit 5-10 Gew% Treibgas beaufschlagt. Alternativ können andere geeignete Container aus Glas, Aluminium, oder Kunststoff verwendet werden. Der abgefüllte Container wird mit einem handelsüblichen Sprühkopf verschlossen. Als Treibgase werden insbesondere Mischungen von Propan und Butan verwendet. Alternativ können auch Dimethylether, Ethylmethylether, Stickstoff, CO₂ oder N₂O verwendet werden. Durch Betätigung des Sprühkopfes können jeweils Einzeldosen von 25 - 200 mg der Sprühformulierung abgegeben werden.
17. Die Zusammensetzung gemäß Beispiel 7 wird in einem in-vitro Modell (hairless mouse skin) getestet, wie beschrieben in Hinz et al., The Journal of Investigative Dermatology, Vol. 93, No. 1, July 1989 oder in Schurad et al. Drug Development and Industrial Pharmacy, Vol. 31, 2005, p. 505-513. Der transdermale Flux des Wirkstoffes beträgt zwischen 40 und 60 µg/cm²/h. Der kumulative Flux in demselben in vitro Modell über 4 Stunden beträgt 160 bis 240 µg/cm²
18. Die Zusammensetzung gemäß Beispiel 9 wird in einem in-vitro Modell (hairless mouse skin) (s.o.) getestet. Der transdermale Flux des Wirkstoffes beträgt zwischen 35 und 60 µg/cm²/h. Der kumulative Flux in demselben in vitro Modell über 4 Stunden beträgt 140 bis 250 µg/cm².

### Zitierte Patentliteratur

US 4303066A
DE 102008060904A1
EP 1 654 013 B1
DE20221244 U1
DE 2343923
DE 2924042
WO 2007/077029A1
US 2016/361271

## Patentansprüche

1. Wirkstoff enthaltendes Sprühpflaster, enthaltend
mindestens ein bei Raumtemperatur leicht verdampfbares Lösungsmittel, mindestens einen pharmazeutischen Wirkstoff, ausgewählt aus Cannabidiol und Tetrahydrocannabidiol,
mindestens ein Acrylpolymer und/oder ein Urethanpolymer, welches nach Verdampfen des Lösemittels einen transparenten, atmungsaktiven Film auf der Haut bildet,
wobei das Acrylpolymer ausgewählt aus Methylprop-2-enoic acid-N,N-dimethylmethanamine (2/1) (Eudragit^{®} RURS) oder ein anionisches Copolymer mit Vinylacetat/Crotonsäure (Luviset CA-66) ist, welches mit geeigneten Aminoalkanolen vorzugsweise mit Aminomethylpropanol neutralisiert ist
und wobei das Urethanpolymer ausgewählt ist aus Polyurethan-4 (Avalur UR 450), Polyurethan-6 (Avalur Flex-6) oder Polyurethane-14 und AMP-Acrylat Copolymer (DynamX)^{®} oder ein anionisches Polymer mit endständiger Säuregruppe ist (Luviset P.U.R.), welches mit organischen oder anorganischen Aminoalkoholen wie z.B. mit Triethanolamin neutralisiert ist,
**dadurch gekennzeichnet, dass** die Sprühlösung zusätzlich mindestens einen komplexierenden Hilfsstoff enthält, wobei als komplexierender Hilfsstoff α-, ß- oder y-Cyclodextrin, substituierte Cyclodextrine, Lecithine, Polysorbate, Glycocholsäure und/oder Pflanzenöle enthalten ist.

2. Wirkstoff enthaltendes Sprühpflaster, gemäß Anspruch 1, wobei als leicht verdampfbare Lösungsmittel Ethanol, Isopropanol, Ethylacetat, Aceton und deren Gemische enthalten sind.

3. Sprühpflaster nach Anspruch 1 oder 2 **dadurch gekennzeichnet, dass** die Formulierungen übliche Weichmacher im Bereich von 0,5 bis 2,5% (w/w) enthalten.

4. Sprühpflaster nach Anspruch 1-3 **dadurch gekennzeichnet, dass** als komplexierende Hilfsstoffe Cyclodextrine, substituierte Cyclodextrine, vorzugsweise 2-Hydroxy-propyl-β-Cyclodextrin (β-HPCD), im Konzentrationsbereich 2-15% (w/w) enthalten sind.

5. Sprühpflaster nach Anspruch 1-4 **dadurch gekennzeichnet, dass** als komplexierende Hilfsstoffe Polysorbat 80, Glycocholsäure und/oder Soja-Lecithin insbesondere im molaren Verhältnis 1:1 bis 1:2 und im Konzentrationsbereich 2-15% (w/w) enthalten sind
oder dass als komplexierende Hilfsstoffe MCT-Öl oder Kokosöl im Konzentrationsbereich 5-15% (w/w) enthalten sind.

6. Sprühpflaster nach Anspruch 1-5 **dadurch gekennzeichnet, dass** Hilfsstoffe enthalten sein können, welche die Permeation von Stoffen durch die Haut verbessern, insbesondere das Natriumsalz der Ölsäure, Isopropylmyristat sowie Kombinationen dieser beiden Verbindungen im Konzentrationsbereich zwischen 0,5 bis 5% (w/w).

7. Sprühpflaster nach Anspruch 1-6, **gekennzeichnet durch** einen Gehalt an feuchthaltenden Hilfsstoffen im Konzentrationsbereich zwischen 0,25 und 10 % (w/w) und mit einem Gewicht pro Sprühstoß von 50 bis 150 mg (Feuchtgewicht).

8. Sprühpflaster nach Anspruch 1-7 **dadurch gekennzeichnet, dass** die Sprühformulierung Menthol, Vanillin, Zimt- und Orangenöl insbesondere R-(+)-Limonen sowie deren Kombinationen im Konzentrationsbereich 0,1 bis 10% (w/w) enthält.

9. Sprühpflaster nach Anspruch 1-8 **dadurch gekennzeichnet, dass** die zur Anwendung verwendeten Pumpspraybehälter mit einem Sprühstoß 25 - 200 mg der Sprühformulierung, vorzugsweise 50 - 100 mg an die Haut abgeben und darin einen Feststoffanteil aus Wirkstoffen, Arzneistoffen, Aromastoffen und nicht flüchtigen Hilfsstoffen von 25 - 50% aufweisen.

## Claims

1. A spray patch comprising an active component, comprising
at least one solvent which is readily vaporizable at room temperature,
at least one pharmaceutical active component, selected from cannabidiol and tetrahydrocannabidiol,
at least one acrylic polymer and/or one urethane polymer forming a transparent breathable film on the skin after vaporization of the solvent,
the acrylic polymer being selected from methylprop-2-enoic acid-N,N-dimethylmethanamine (2/1) (Eudragit^{®} RUAS) or an anionic copolymer with vinyl acetate/crotonic acid (Luviset CA-66) neutralized with suitable aminoalkanols, preferably with aminomethylpropanol, and
the urethane polymer being selected from polyurethane-4 (Avalur UR 450), polyurethane-6 (Avalur Flex-6), or polyurethane-14 and AMP-acrylate copolymer (DynamX)^{®} or an anionic polymer with a terminal acid group (Luviset P.U.R.), which is neutralized with organic or inorganic amino alcohols such as triethanolamine,
**characterized in that** the spray solution additionally contains at least one complexing auxiliary, the complexing auxiliary being α-, β-, or y-cyclodextrin, substituted cyclodextrins, lecithins, polysorbates, glycocholic acid, and/or vegetable oils.

2. The spray patch comprising an active component according to claim 1, wherein the included readily vaporizable solvents are ethanol, isopropanol, ethyl acetate, acetone, and mixtures thereof.

3. The spray patch according to claim 1 or 2, **characterized in that** the formulations contain conventional plasticizers in the range from 0.5 to 2.5% (w/w).

4. The spray patch according to claim 1 to 3, **characterized in that** the complexing auxiliaries are cyclodextrins, substituted cyclodextrins, preferably 2-hydroxy-propyl-β-cyclodextrin (β-HPCD) in the concentration range from 2 to 15% (w/w).

5. The spray patch according to claim 1 to 4, **characterized in that** the complexing auxiliaries are polysorbate 80, glycocholic acid, and/or soy lecithin, in particular in a molar ratio from 1:1 to 1:2 and in the concentration range from 2 to 15% (w/w), or that the complexing auxiliaries are MCT oil or coconut oil in a concentration range from 5 to 15% (w/w).

6. The spray patch according to claim 1 to 5, **characterized in that** it may contain auxiliaries, which enhance the permeation of substances through the skin, in particular the sodium salt of oleic acid, isopropyl myristate, and combinations of these two compounds in the concentration range from 0.5 to 5% (w/w).

7. The spray patch according to claim 1 to 6, **characterized in** a content of moisturizing auxiliaries in the concentration range from 0.25 to 10% (w/w) and with a weight per spray jet of 50 to 150 mg (wet weight).

8. The spray patch according to claim 1 to 7, **characterized in that** the spray formulation contains menthol, vanillin, cinnamon, and orange oil, in particular R-(+) limonene, and combinations thereof in the concentration range from 0.1 to 10% (w/w).

9. The spray patch according to claim 1 to 8, **characterized in that** the pump spray containers used for the application deliver a spray jet of 25 - 200 mg of the spray formulation, preferably 50 - 100 mg, to the skin and have a solid content of active components, medicinal substances, aromatic substances, and non-volatile auxiliaries of 25 - 50%.

## Revendications

1. Pansement sous forme de spray comprenant un agent actif, comprenant
au moins un solvant aisément vaporisable à la température ambiante,
au moins un agent actif pharmaceutique, choisi à partir de cannabidiol et tétrahydrocannabidiol,
au moins un polymère acrylique et/ou un polymère d'uré-thane, qui forme un film transparent, imper-respirant sur la peau après vaporisation du solvant,
le polymère acrylique étant choisi à partir d'acide méthyl-2-propénoïque-N,N-diméthylméthanamine (2/1) (Eudragit^{®} RUAS) ou un copolymère anionique avec acétate de vinyle/acide crotonique (Luviset CA-66), qui est neutralisé par des aminoalcanols appropriés, de préférence par aminométhylpropanol, et
le polymère d'uréthane étant choisi à partir de polyuréthane-4 (Avalur UR 450), polyuréthane-6 (Avalur Flex-6), ou polyuréthane-14 et AMP-copolymère d'acrylates (DynamX)^{®} ou d'un polymère anionique avec un groupe acide terminal (Luviset P.U.R.), qui est neutralisé par des alcools aminés organiques ou inorganiques comme triétha-nolamine,
**caractérisé en ce que** la solution spray en outre contient au moins un adjuvant complexant, l'adjuvant complexant étant une α-, β- ou y-cyclodextrine, des cyclodextrines substituées, lécithines, polysorbates, de l'acide glycocholique, et/ou des huiles végétales.

2. Pansement sous forme de spray comprenant un agent actif selon la revendication 1, dans lequel de l'éthanol, isopropanol, acétate d'éthyle, acétone, et des mélanges de ceux-ci sont compris comme des solvants aisément vaporisables.

3. Pansement sous forme de spray selon la revendication 1 ou 2, **caractérisé en ce que** les formulations comprennent des agents plastifiants dans la gamme de 0,5 à 2,5% en poids.

4. Pansement sous forme de spray selon la revendication 1 à 3, **caractérisé en ce que** les adjuvants complexants sont des cyclodextrines, cyclodextrines substituées, de préférence 2-hydroxypropyl-β-cyclodextrine (β-HPCD) dans la gamme de concentration de 2 à 15% en poids.

5. Pansement sous forme de spray selon la revendication 1 à 4, **caractérisé en ce que** les adjuvants complexants sont du polysorbate 80, acide glycocholique, et/ou soja lécithine, en particulier dans le ratio molaire 1:1 à 1:2 et dans la gamme de concentration de 2 à 15% en poids, ou que les adjuvants complexants sont de l'huile MCT ou de l'huile de noix de coco dans la gamme de concentration de 5 à 15% en poids.

6. Pansement sous forme de spray selon la revendication 1 à 5, **caractérisé en ce que** des adjuvants peuvent être compris, qui améliorent la perméation de substances à travers la peau, en particulier le sel de sodium de l'acide oléique, myristate d'isopropyl et des combinaisons des ces deux composés dans la gamme de concentration de 0,5 à 5% en poids.

7. Pansement sous forme de spray selon la revendication 1 à 6, caractérisé en une teneur en adjuvants hydratants dans la gamme de concentration de 0,25 à 10% en poids et un poids par un jet de spray de 50 à 150 mg (poids humide).

8. Pansement sous forme de spray selon la revendication 1 à 7, **caractérisé en ce que** la formulation du spray contient du menthol, vanilline, huile de cannelle et d'orange, en particulier R-(+) limonène, et des combinaisons de ceux-ci dans la gamme de concentration de 0,1 à 10% en poids.

9. Pansement sous forme de spray selon la revendication 1 à 8, **caractérisé en ce que** les flacons à pompe utilisés pour l'application dégagent un jet de spray de 25 à 200 mg de la formulation de spray, de préférence 50 à 100 mg, à la peau et ont une teneur en solides d'agents actifs, médicaments, substances aromatiques et adjuvants non volatiles de 25 - 50%.
